(19) Europäisches Patentamt
European Patent Office
Office européen des brevets

(11) **EP 4 309 507 A1**

(12) **EUROPEAN PATENT APPLICATION**
published in accordance with Art. 153(4) EPC

(43) Date of publication:
**24.01.2024 Bulletin 2024/04**

(51) International Patent Classification (IPC):
***A23F 3/16*** (2006.01)          ***C12N 1/20*** (2006.01)

(21) Application number: **22771353.4**

(52) Cooperative Patent Classification (CPC):
**A23F 3/16; C12N 1/20**

(22) Date of filing: **14.03.2022**

(86) International application number:
**PCT/JP2022/011200**

(87) International publication number:
**WO 2022/196607 (22.09.2022 Gazette 2022/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.03.2021 JP 2021046535
11.10.2021 JP 2021166751**

(71) Applicant: **KIRIN HOLDINGS KABUSHIKI KAISHA
Nakano-ku, Tokyo 164-0001 (JP)**

(72) Inventors:
• **SUZUKI, Mihoko
  Tokyo 164-0001 (JP)**
• **YOTSUMOTO, Yuko
  Tokyo 164-0001 (JP)**

(74) Representative: **Hoffmann Eitle
Patent- und Rechtsanwälte PartmbB
Arabellastraße 30
81925 München (DE)**

(54) **CONTAINER-PACKED TEA BEVERAGE WITH REDUCED LIQUID COLOR DEGRADATION DURING HEAT STERILIZATION, AND METHOD FOR MANUFACTURING SAME**

(57) An object of the present invention is to provide, for example, a packaged tea beverage in which liquid color degradation during heat sterilization is suppressed, and a production method therefor. The method of the present invention comprises the step of including lactic acid bacteria in the production of a heat-sterilized packaged tea beverage having a tea polyphenol concentration of 10 to 200 mg/100 mL and a pH of 4.5 to 7.5.

EP 4 309 507 A1

## Description

### Technical Field

The present invention relates to a packaged tea beverage in which liquid color degradation during heat sterilization is suppressed, and a production method therefor.

### Background Art

Food and drink products having a health-promoting effect have received growing attention in recent years because of increasing health consciousness. Among others, tea beverages such as green tea beverages or black tea beverages contain a health-supporting ingredient such as catechin or theanine, and there are increasing consumers' demands therefor. Many packaged tea beverages have been launched, because the tea beverages can be drunk, regardless of locations or times.

Packaged green tea beverages are known to cause a phenomenon in which the color of green tea is changed from light green color to red-brown color due to the oxidation of components in the green tea beverages during preservation. This phenomenon is generally called "browning". This browning phenomenon occurs in the production of tea beverages, particularly, during heat sterilization of tea extract liquids, and also during distribution or storage, and not only reduces the value of commodity but causes unpleasant taste or smell. In packaged black tea beverages as well, liquid color degradation is known to occur during heat sterilization and the like. Hence, there has been a demand for a method for suppressing liquid color degradation in tea beverages such as green tea beverages or black tea beverages.

As for methods for suppressing browning in the course of production and after production of green tea beverages, for example, patent document 1 discloses a method comprising, in a method for producing a green tea beverage, performing the extraction of green tea using warm water of 40 to 80°C containing sugar ester, adding sodium ascorbate during or after the extraction, and replacing residual air in a package with nitrogen gas after filling of a green tea extract liquid into the package; patent document 2 discloses a method of adding trehalose to a green tea beverage; and patent document 3 discloses a method of adding L-ascorbic acid 2-glucoside to a green tea beverage.

In addition, as for techniques of exploiting lactic acid bacteria in food and drink products, patent document 4 discloses a method for promoting the proliferation of lactic acid bacteria to be grown and improving the viability of the lactic acid bacteria in a product by adding dead bacteria of the lactic acid bacteria or a cultured product containing dead bacteria of the lactic acid bacteria during culture of the lactic acid bacteria to be grown; and patent document 5 discloses a method for improving the richness (good taste) of coffee by blending dead bacteria of lactic acid bacteria or the like into a packaged coffee beverage.

However, it has not been known so far that lactic acid bacteria contained in a packaged tea beverage can suppress liquid color degradation during heat sterilization of the tea beverage.

### Prior Art Documents

### Patent Documents

Patent Document 1: Japanese Patent No. 1571801
Patent Document 2: Japanese unexamined Patent Application Publication No. 2001-112414
Patent Document 3: Japanese unexamined Patent Application Publication No. 2007-60972
Patent Document 4: Japanese unexamined Patent Application Publication No. 2008-5811
Patent Document 5: Japanese unexamined Patent Application Publication No. 2019-122316

### Summary of the Invention

### Object to be Solved by the Invention

An object of the present invention is to provide, for example, a packaged tea beverage in which liquid color degradation during heat sterilization is suppressed, and a production method therefor.

### Means to Solve the Object

The present inventors have conducted diligent studies to attain the object of the present invention, and consequently completed the present invention by finding that allowing lactic acid bacteria to be contained in the production of a packaged tea beverage can suppress liquid color degradation of the tea beverage during heat sterilization, whereby the object can be attained.

Specifically, the present invention relates to:

(1) a heat-sterilized packaged tea beverage having a tea polyphenol concentration of 10 to 200 mg/100 mL and a pH of 4.5 to 7.5, wherein the packaged tea beverage comprises lactic acid bacteria;

(2) the packaged tea beverage according to (1), wherein a content of the lactic acid bacteria is 10 billion or more bacteria/100 mL;

(3) the packaged tea beverage according to (1) or (2), wherein the lactic acid bacteria are bacteria of the genus *Lactococcus;*

(4) the packaged tea beverage according to any one of (1) to (3), wherein the lactic acid bacteria are *Lactococcus lactis* subsp. *lactis* JCM5805;

(5) the packaged tea beverage according to any one of (1) to (4), wherein the packaged tea beverage is a packaged green tea beverage or a packaged black tea beverage;

(6) a method for producing a packaged tea beverage, comprising a step of including lactic acid bacteria in the production of a heat-sterilized packaged tea beverage having a tea polyphenol concentration of 10 to 200 mg/100 mL and a pH of 4.5 to 7.5;

(7) the method for producing a packaged tea beverage according to (6), wherein the step of including lactic acid bacteria is carried out at any stage before heat sterilization in the production of the packaged tea beverage;

(8) a method for suppressing liquid color degradation during heat sterilization of a packaged tea beverage, comprising a step of including lactic acid bacteria in the production of a heat-sterilized packaged tea beverage having a tea polyphenol concentration of 10 to 200 mg/100 mL and a pH of 4.5 to 7.5; and

(9) the method for suppressing liquid color degradation during heat sterilization of a packaged tea beverage according to (8), wherein the step of including lactic acid bacteria is carried out at any stage before heat sterilization in the production of the packaged tea beverage.

### Effect of the Invention

The present invention can provide, for example, a packaged tea beverage in which liquid color degradation during heat sterilization is suppressed, and a production method therefor.

### Brief Description of Drawing

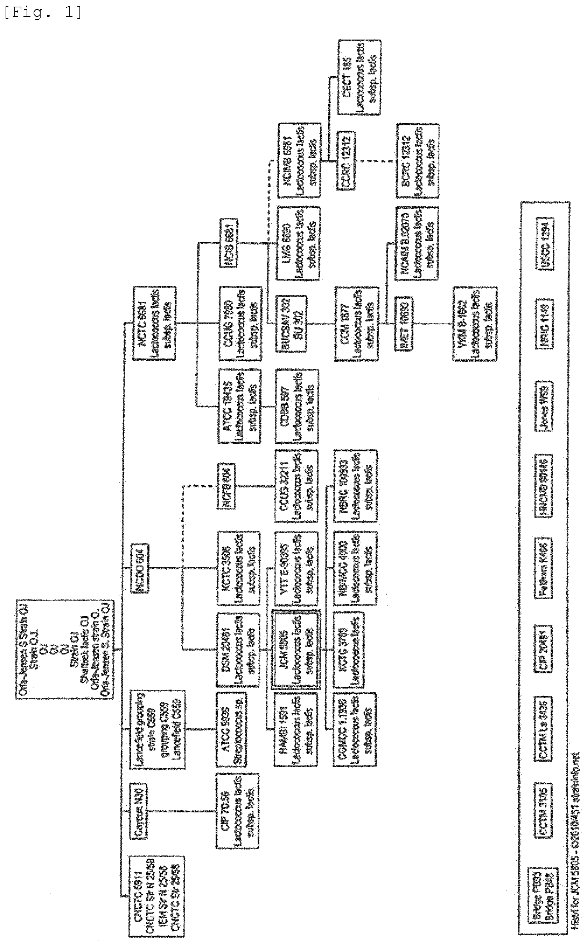

[Figure 1] Figure 1 is a diagram showing the relationship between a *Lactococcus lactis* subsp. *lactis* JCM5805 strain and strains equivalent to the strain (strains derived from the strain and strains from which the strain is derived).

### Mode of Carrying Out the Invention

The present invention includes embodiments such as:

[1] a heat-sterilized packaged tea beverage having a tea polyphenol concentration of 10 to 200 mg/100 mL and a pH of 4.5 to 7.5, wherein the packaged tea beverage comprises lactic acid bacteria;

[2] a method for producing a packaged tea beverage, comprising the step of including lactic acid bacteria in the production of a heat-sterilized packaged tea beverage having a tea polyphenol concentration of 10 to 200 mg/100 mL and a pH of 4.5 to 7.5; and

[3] a method for suppressing liquid color degradation during heat sterilization of a packaged tea beverage, comprising the step of including lactic acid bacteria in the production of a heat-sterilized packaged tea beverage having a tea polyphenol concentration of 10 to 200 mg/100 mL and a pH of 4.5 to 7.5.

(Packaged tea beverage)

The packaged tea beverage according to the present invention is a heat-sterilized packaged tea beverage having a tea polyphenol concentration of 10 to 200 mg/100 mL and a pH of 4.5 to 7.5, wherein the packaged tea beverage comprises lactic acid bacteria.

Examples of the "tea beverage" according to the present invention include, but are not particularly limited to, an unfermented tea (e.g., green tea) beverage, a fermented tea (e.g., black tea) beverage, and a semi-fermented tea (e.g., oolong tea) beverage, and a blend tea beverage of a portion or the whole of these. Among them, a green tea beverage, an oolong tea beverage, and a black tea beverage are preferred. Among them, a green tea beverage and a black tea beverage are more preferred from the viewpoint of enjoying more effects of the present invention.

(Tea polyphenol concentration)

The tea polyphenol concentration of the packaged tea beverage according to the present invention is not particularly limited. The tea polyphenol concentration is preferably 10 to 200 mg/100 mL, more preferably 40 to 100 mg/100 mL, from the viewpoint of larger change ascribable to liquid color degradation during heat sterilization and enjoying more effects of suppressing liquid color degradation according to the present invention, and from the viewpoint of favorable flavor of the packaged tea beverage. The tea polyphenol concentration of the packaged tea beverage can be adjusted by adjusting the amount of the tea leaves used in the production of the tea beverage, or the amount of the concentrate or purified product of the tea extract liquid used.

The tea polyphenol concentration of the packaged tea beverage according to the present invention can be measured by use of an official method (iron tartrate absorbance method) described in "Explanation of Analytical Manual for Standard Tables of Food Composition in Japan, 5th edition" edited by Japan Food Research Laboratories (Japan Food Research Laboratories ed., Chuohoki Publishing Co., Ltd., July 2001, p. 252) which is a standard for evaluating the amount of polyphenol in teas. In this measurement method, absorbance (540 nm) can be measured as to a purple component formed through the reaction of polyphenol in a liquid with an iron tartrate reagent, thereby achieving quantification using a calibration curve prepared with ethyl gallate as a standard substance. The quantified value thus obtained is multiplied by 1.5, and the resulting value can be used as the amount of tea polyphenol.

The measurement of the tea polyphenol concentration may employ a Folin-Ciocalteu method, a Folin-Denis method, or the like in addition to the iron tartrate absorbance method described above. The iron tartrate absorbance method is preferred when the iron tartrate absorbance method is suitable.

(pH)

The pH of the packaged tea beverage according to the present invention is not particularly limited and is, for example, 4.5 to 7.5, preferably 6 to 7.3.

The pH of the tea beverage can be measured by a routine method using a pH meter.

(Lactic acid bacteria)

The packaged tea beverage according to the present invention contains lactic acid bacteria.

The lactic acid bacteria for use in producing the packaged tea beverage according to the present invention are not particularly limited as long as they are lactic acid bacteria. The lactic acid bacteria may be live bacteria of the lactic acid bacteria or dead bacteria of the lactic acid bacteria and are preferably dead bacteria of the lactic acid bacteria. Such dead bacteria of the lactic acid bacteria may be a dried product or a non-dried product and is preferably a dried product of the lactic acid bacteria, more preferably a dry powder of the lactic acid bacteria, from the viewpoint of preservation stability for example.

A method for obtaining the dry powder is not particularly designated. The dry powder can be obtained by a treatment such as freeze drying, hot air drying, or spray drying, and the powder is preferably obtained by spray drying.

In the case of using live bacteria of the lactic acid bacteria in producing the packaged tea beverage according to the present invention, basically, most of the lactic acid bacteria also become dead bacteria by a heat sterilization step.

The "lactic acid bacteria" are a generic name for all bacteria taxonomically approved as lactic acid bacteria, and are not limited by a genus, a species, a strain, or the like. Examples of such "lactic acid bacteria" include bacteria that produce a large amount of lactic acid (preferably lactic acid in an amount of 50% or more of consumed sugar) by the lactic acid fermentation of sugar, and include bacteria of the genus *Lactobacillus*, bacteria of the genus *Streptococcus*, bacteria of the genus *Lactococcus*, bacteria of the genus *Leuconostoc*, bacteria of the genus *Pediococcus*, and bacteria of the genus *Enterococcus*.

The genus or species of the lactic acid bacteria used in the present invention is not particularly limited. The lactic acid bacteria are one or more types of bacteria selected from the group consisting of bacteria of the genus *Lactobacillus*, bacteria of the genus *Streptococcus*, bacteria of the genus *Lactococcus*, bacteria of the genus *Leuconostoc*, bacteria of the genus *Pediococcus*, and bacteria of the genus *Enterococcus*, preferably one or more types of bacteria selected from the group consisting of bacteria of the genus *Lactococcus*, more preferably one or more types of bacteria selected from the group consisting of *Lactococcus lactis* subsp. *lactis* (hereinafter, also simply referred to as "*Lactococcus lactis*").

In more specific preferred aspect, the lactic acid bacteria according to the present invention are one or more types of bacteria selected from the group consisting of *Lactobacillus acidophilus*, *Lactobacillus delbrueckii* subsp. *bul-*

*garicus, Lactobacillus delbrueckii* subsp. delbrueckii, *Lactobacillus delbrueckii* subsp. *lactis, Lactobacillus casei, Lactobacillus paracasei, Lactobacillus gasseri, Lactobacillus helveticus, Lactobacillus johnsonii, Lactobacillus plantarum, Lactobacillus brevis,* Lactobacillus casei subsp. *rhamnosus, Lactobacillus pentosus, Lactobacillus fermentum, Streptococcus salivarius* subsp. *thermophilus* (hereinafter, also simply referred to as *"Streptococcus thermophilus"*)*, Lactococcus lactis* subsp. *lactis* (hereinafter, also simply referred to as *"Lactococcus lactis"*)*, Lactococcus lactis* subsp. *lactis biovar diacetylactis, Lactococcus lactis* subsp. *cremoris, Lactococcus raffinolactis, Lactococcus piscium, Lactococcus plantarum, Lactococcus garvieae, Lactococcus lactis* subsp. *hordniae, Leuconostoc mesenteroides* subsp. *cremoris, Leuconostoc lactis, Pediococcus damnosus, Pediococcus pentosaceus, Pediococcus acidilactici, Enterococcus faecalis,* and *Enterococcus faecium,* preferably one or more types of bacteria selected from the group consisting of *Lactobacillus acidophilus, Lactobacillus delbrueckii* subsp. *bulgaricus, Lactobacillus delbrueckii* subsp. *lactis, Lactobacillus delbrueckii* subsp. *delbrueckii, Lactobacillus casei, Lactobacillus paracasei, Lactobacillus gasseri, Lactobacillus helveticus, Lactobacillus johnsonii, Lactobacillus plantarum, Lactobacillus brevis, Lactobacillus casei* subsp. *rhamnosus, Lactobacillus pentosus, Lactobacillus fermentum, Streptococcus salivarius* subsp. *thermophilus, Lactococcus lactis* subsp. *lactis, Lactococcus lactis* subsp. *lactis biovar diacetylactis, Lactococcus lactis* subsp. *cremoris, Lactococcus raffinolactis, Lactococcus piscium, Lactococcus plantarum, Lactococcus garvieae, Lactococcus lactis* subsp. *hordniae, Leuconostoc mesenteroides* subsp. *cremoris,* and *Leuconostoc lactis,* more preferably one or more types of bacteria selected from the group consisting of *Lactococcus lactis, Lactococcus lactis* subsp. *lactis biovar diacetylactis, Lactococcus lactis* subsp. *cremoris, Lactococcus raffinolactis, Lactococcus piscium, Lactococcus plantarum, Lactococcus garvieae,* and *Lactococcus lactis* subsp. *hordniae,* still more preferably *Lactococcus lactis,* even more preferably one or more types of bacteria selected from the group consisting of *Lactococcus lactis* JCM5805, *Lactococcus lactis* JCM20101, *Lactococcus lactis* NBRC12007, and *Lactococcus lactis* NRIC1150, particularly preferably *Lactococcus lactis* JCM5805. A suitable form of such a *Lactococcus lactis* JCM5805 strain is preferably dead bacteria of the JCM5805 strain, more preferably a dried product of dead bacteria of the JCM5805 strain, still more preferably a dry powder of dead bacteria of the JCM5805 strain, even more preferably a spray-dried powder of dead bacteria of the JCM5805 strain.

The "JCM5805 strain" according to the present invention also includes a bacterial strain equivalent to the JCM5805 strain as long as the effects of the present invention can be obtained. In this context, the equivalent bacterial strain refers to a bacterial strain derived from the JCM5805 strain (bacterial strain A), a bacterial strain from which the JCM5805 strain is derived (bacterial strain B), or a progeny bacterial strain of the bacterial strain A or the bacterial strain B mentioned above. The equivalent bacterial strain may be preserved in other bacterial strain preservation institutes. Figure 1 shows bacterial strains derived from the JCM5805 strain and bacterial strains from which the JCM5805 strain is derived. The bacterial strains equivalent to the JCM5805 strain described in Figure 1 can also be used as the "JCM5805 strain" according to the present invention as long as the effects of the present invention can be obtained.

Alternatively, the "lactic acid bacteria" according to the present invention may be a *Lactobacillus paracasei* KW3110 strain (hereinafter, referred to as a "KW3110 strain"). It is preferred that the lactic acid bacteria should not be the KW3110 strain.

The lactic acid bacteria used in the present invention can be obtained from a deposition institute such as American Type Culture Collection (USA) . Alternatively, a commercially available starter culture may be used as the lactic acid bacteria used in the present invention.

A method for preparing the lactic acid bacteria used in the present invention is not particularly limited. Examples of the method for preparing the live bacteria of the lactic acid bacteria can include a method of collecting bacteria by filtration, centrifugation, or the like from a medium in which the lactic acid bacteria have been cultured. Examples of the method for preparing the dead bacteria of the lactic acid bacteria can include a method of sterilizing a medium in which the lactic acid bacteria has been cultured, and then collecting bacteria by filtration, centrifugation, or the like, and a method of collecting bacteria by filtration, centrifugation, or the like from a medium in which the lactic acid bacteria has been cultured, followed by sterilization. If necessary, a drying treatment or a homogenization treatment can be further performed.

An approach for the sterilization is not particularly limited. Not only heating but a common approach of killing bacteria, such as ultraviolet ray or $\gamma$ ray irradiation, can be used.

In the present invention, the content of the lactic acid bacteria in the packaged tea beverage is not particularly limited as long as the effects of the present invention can be obtained. The number of bacteria of the lactic acid bacteria per 100 mL of the packaged tea beverage is, for example, "6 billion bacteria/100 mL" or more, preferably "10 billion bacteria/100 mL" or more, more preferably "15 billion bacteria/100 mL" or more, still more preferably "30 billion bacteria/100 mL" or more, even more preferably "150 billion bacteria/100 mL" or more, further preferably "450 billion bacteria/100 mL" or more, from the viewpoint of more largely suppressing liquid color degradation during heat sterilization, for example.

The upper limit thereof is "1 trillion bacteria/100 mL" or less, preferably "750 billion bacteria/100 mL" or less, more preferably "500 billion bacteria/100 mL" or less.

These upper limit values and lower limit values can be arbitrarily combined.

More specifically, a suitable content of the lactic acid bacteria in the packaged tea beverage is, for example, 6 to 500 billion bacteria/100 mL, preferably 10 to 480 billion bacteria/100 mL, more preferably 16 to 450 billion bacteria/100 mL, still more preferably 16 to 300 billion bacteria/100 mL, even more preferably 16 to 150 billion bacteria/100 mL.

The number of bacteria of the lactic acid bacteria can be counted by direct microscopic examination, particle electrical sensing zone, PCR, flow cytometry, or the like.

The number of bacteria per g of a dried product of the bacteria of the lactic acid bacteria can be measured by the counting method mentioned above, and the amount of the JCM5805 strain blended into the packaged tea beverage can thereby be adjusted.

The number of bacteria of the lactic acid bacteria in the packaged tea beverage can be counted by direct microscopic examination, flow cytometry, or the like. More accurately, a method of taking into consideration the influence of particles derived from other raw materials in the beverage can be used.

(Production of packaged tea beverage according to present invention)

The packaged tea beverage according to the present invention can be produced by a method comprising the step of including lactic acid bacteria in the production of a heat-sterilized packaged tea beverage having a tea polyphenol concentration of 10 to 200 mg/100 mL and a a pH of 4.5 to 7.5. The production method of the present invention can employ a general method for producing packaged tea beverages except that lactic acid bacteria are included. Examples of such a general production method include a method of preparing a tea extract liquid, and producing a packaged tea beverage through a compounding step, a filling step, and a heat sterilization step. In the beverage production of the present invention, an additive for a beverage that is usually used in the formulation of usual tea beverages may be added, and the timing of addition of such an additive is not particularly limited. For more details of the general method for producing packaged tea beverages mentioned above, see, for example, "Soft Drinks, revised new edition" (Korin Co., Ltd.).

Examples of the "step of including lactic acid bacteria " according to the present invention preferably include adding the lactic acid bacteria such that the tea beverage contains the lactic acid bacteria, and include adding the lactic acid bacteria to the tea extract liquid itself as well as adding the lactic acid bacteria to water for use in the preparation of the tea extract liquid.

In the present invention, the timing of including the lactic acid bacteria (preferably adding the lactic acid bacteria) can be any stage in the production process of the packaged tea beverage and is preferably any stage before heat sterilization as long as the effects of the present invention can be obtained. Specifically, for example, the lactic acid bacteria may be included in the tea extract liquid in a tea extraction step, the lactic acid bacteria may be included in water in a tea extraction step, the lactic acid bacteria may be included in the tea extract liquid in a compounding step, or the lactic acid bacteria may be included in the tea extract liquid immediately before or immediately after a filling step into a package.

The "tea extract liquid" according to the present invention means an extract liquid that is obtained by subjecting tea leaves (including a pulverized product of tea leaves such as powdered green tea) to an extraction treatment. Examples of the tea extract liquid used in the present invention include an extract liquid itself from tea leaves (tea leaf extract liquid), and diluted liquids of processed products (e.g., concentrated liquid extracts and powder extracts) thereof. A conventional tea extract raw material (preferably a green tea extract raw material, a black tea extract raw material, and/or an oolong tea extract raw material) that is used in the production of tea beverages can be appropriately selected without particular limitations. In the present specification, examples of the "tea extract liquid" include an unfermented tea extract liquid (e.g., a green tea extract liquid), a fermented tea extract liquid (e.g., a black tea extract liquid), and a semi-fermented tea extract liquid (e.g., an oolong tea extract liquid). Among them, a green tea extract liquid, a black tea extract liquid, and an oolong tea extract liquid are preferred. Among them, a green tea extract liquid and a black tea extract liquid are more preferred. In the present invention, the extraction treatment also includes, for example, a form such as a treatment of dissolving a pulverized product of tea leaves such as powdered green tea as long as a tea component is eluted into an extracting solvent.

Tea leaves belonging to a tea tree *Camellia sinensis* var. which is an evergreen tree of the family *Theaceae* can be used as tea leaves for use in the preparation of the tea extract liquid. The tea leaves for use in the production of the tea beverage of the present invention is not particularly limited as long as the effects of the present invention are exerted. Not only tea leaves of unfermented tea typified by green tea such as middle-quality green tea, high-quality green tea, powdered green tea, pot-roasted tea, deep steamed tea, coarse tea, or roasted green tea but tea leaves of semi-fermented tea such as oolong tea, tea leaves of fermented tea such as black tea, or the like can be used. Examples of the tea leaves used in the present invention preferably include green tea leaves and black tea leaves. In the present invention, plural types of raw materials and tea leaves may be used.

A method for the extraction treatment of the tea leaves is not particularly limited. Various extraction methods that are generally used in the food processing field can be used and encompass, for example, solvent extraction, air current extraction, and squeeze extraction. If necessary, solid-liquid separation such as precipitation or filtration, or a treatment such as concentration, centrifugation, drying (e.g., spray drying and freeze drying), or powdering may be further carried out.

In this context, the extracting solvent for use in solvent extraction is desirably water (e.g., hard water, soft water, ion exchange water, and natural water). The amount of the extracting solvent can be appropriately selected by those skilled in the art, and is not particularly limited. When the extracting solvent is, for example, water, the amount thereof is 1 to 100 times the amount (mass) of the tea leaves.

The extraction temperature and the extraction time can be appropriately selected by those skilled in the art, and are not particularly limited. When the extracting solvent is, for example, water, the temperature and time thereof are 10 to 120°C and 1 minute to 12 hours.

One example of the extraction treatment includes a method of dipping and stirring tea leaves in water at 0 to 90°C for 1 minute to 24 hours, and then filtering or centrifuging the tea leaves. In this context, conditions, such as a temperature and a time, for extraction are not particularly limited and can be arbitrarily selected and set by those skilled in the art depending on the type or amount of the tea leaves.

In the preparation of the tea extract liquid, a concentrate or a purified product of the tea extract liquid, such as a tea extract or a tea powder, may be used, and a commercially available product, for example, POLYPHENON (manufactured by Mitsui Norin Co., Ltd.), Sunphenon (manufactured by Taiyo Kagaku Co., Ltd.), or Teaflan (manufactured by Itoen, Ltd.) can be used. Such a tea concentrate (preferably a green tea concentrate, a black tea concentrate, and/or an oolong tea concentrate, etc.) or a tea purified product (preferably a green tea purified product, a black tea purified product, and/or an oolong tea purified product, etc.) may be used alone as it is or after being dissolved in or diluted with water, or plural types thereof may be used as a mixture, or the tea concentrate(s) or the tea purified product (s) may be mixed with the tea extract liquid for use.

An arbitrary raw material other than the tea leaves may be blended in the preparation of the tea extract liquid. The tea leaves or the tea extract liquid used in the present invention may be tea leaves or a tea extract liquid fermented with lactic acid bacteria as long as the effects of the present invention can be obtained. It is preferred to be tea leaves (preferably green tea leaves, black tea leaves, and/or oolong tea leaves) or a tea extract liquid (preferably a green tea extract liquid, a black tea extract liquid, and/or an oolong tea extract liquid) that is not fermented with lactic acid bacteria.

(Heat sterilization)

The packaged tea beverage according to the present invention is a heat-sterilized packaged tea beverage. The heat sterilization step may be performed before or after filling into a package.

Various heat sterilization methods that are generally used in the food field can be used as a heat sterilization method. Examples thereof include a method using a hot water spray-type, hot water storage-type, or steam-type retort sterilization apparatus or a tube-type or plate-type liquid continuous sterilization apparatus.

Conditions for the heat sterilization according to the present invention are not particularly limited, and usual conditions that are used in the heat sterilization of packaged tea beverages can be used. Such conditions are, for example, conditions with an $F_0$ value of 4 to 40, preferably conditions of 111 to 150°C and a time that attains an $F_0$ value of 4 to 20, because a relatively short time suffices as a sterilization time.

(Optional component)

The packaged tea beverage of the present invention may be supplemented with an additive for beverages that is used in the production of usual beverages, for example, an acidulant, a flavor, a dye, fruit juice, a sweetener (including a high-intensity sweetener), a dairy ingredient, a pulverized product of tea leaves (powdered green tea, dust tea, etc.), and a food additive (e.g., an antioxidant, a preservative, a thickening stabilizer, an emulsifier, a dietary fiber, a pH adjuster, and a bittering agent).

The tea beverage according to the present invention may not contain ascorbic acid and/or an ascorbic acid-derived substance. It is preferred to contain ascorbic acid and/or an ascorbic acid-derived substance. The "ascorbic acid" or the "ascorbic acid-derived substance" according to the present invention is not particularly limited as long as its blending into beverages is acceptable. The "ascorbic acid" according to the present invention includes not only L-ascorbic acid but its isomer (isoascorbic acid, etc.).

The "ascorbic acid-derived substance" according to the present invention includes a salt of ascorbic acid, a derivative of ascorbic acid, and a salt of a derivative of ascorbic acid. Among them, a salt of ascorbic acid is preferred. Examples of the derivative of ascorbic acid include ascorbyl fatty acid ester (e.g., ascorbyl stearate, ascorbyl palmitate, etc.), ascorbyl phosphate, ascorbyl sulfate, and ascorbic acid glycoside.

Examples of the salt of ascorbic acid or its derivative can include: a salt of an alkali metal such as sodium or potassium; a salt of an alkaline earth metal such as calcium, magnesium, or barium; a salt of a basic amino acid such as arginine or lysine; an ammonium salt such as ammonium salt and tricyclohexylammonium salt; and an alkanolamine salt such as monoisopropanolamine salt, diisopropanolamine salt, and triisopropanolamine salt. Among them, an alkali metal salt and an alkaline earth metal salt are preferred.

Specific examples of the salt of ascorbic acid preferably include sodium ascorbate, calcium ascorbate, and sodium isoascorbate. More specific examples of the derivative of ascorbic acid, or its salt include ascorbyl 2,6-dipalmitate, ascorbyl 6-stearate, sodium ascorbyl 2-phosphate, disodium ascorbyl 2-sulfate, ascorbic acid 2-glucoside, ascorbic acid glucosamine, L-dehydroascorbic acid, ascorbyl 6-palmitate, L-ascorbine tetraisopalmitate, ascorbyl tetra-2-hexyldecanoate, and magnesium L-ascorbyl phosphate.

One type of "ascorbic acid" or "ascorbic acid-derived substance" used in the present invention may be used singly, or two or more types thereof may be used in combination. A commercially available product may be used as the "ascorbic acid" or the "ascorbic acid-derived substance".

The amount of the ascorbic acid and/or the ascorbic acid-derived substance according to the present invention used (the content of the ascorbic acid and/or the ascorbic acid-derived substance in the beverage or the amount of the ascorbic acid and/or the ascorbic acid-derived substance added to the beverage) is not particularly limited as long as the effects of the present invention can be obtained. The total concentration of the "ascorbic acid" and the "ascorbic acid-derived substance" based on ascorbic anhydride in the packaged tea beverage is 0.01 to 0.5% by weight, preferably 0.01 to 0.2% by weight, more preferably 0.02 to 0.1% by weight, still more preferably 0.03 to 0.08% by weight.

The ascorbic acid concentration in the tea beverage can be analyzed by use of, for example, HPLC.

(Package)

The tea beverage according to the present invention is a packaged tea beverage. Such a package means a sealed package that can break the contact between contents and outside air. Examples thereof include a metal can, a barrel container, a plastic bottle (e.g., a PET bottle and a cup), a paper container, a bottle, and a pouch container.

(Packaged tea beverage in which liquid color degradation during heat sterilization is suppressed)

The packaged tea beverage of the present invention is a packaged tea beverage in which liquid color degradation during heat sterilization is suppressed. In the present specification, the phrase "liquid color degradation during heat sterilization is suppressed" means a packaged tea beverage in which liquid color degradation during heat sterilization is suppressed as compared with liquid color degradation during heat sterilization of a packaged tea beverage produced by the same production method (including heat sterilization conditions) using the same raw material as in the packaged tea beverage of the present invention except that the lactic acid bacteria are not contained (hereinafter, "also referred to as the control beverage according to the present invention".

In the present invention, the "liquid color degradation" is indicated by color difference $\Delta E^*ab$ of the sample with respect to the nonheated sample. The color difference $\Delta E^*ab$ is a difference in coordinates $L^*$, $a^*$, and $b^*$ in $L^*a^*b^*$ color space and is a color difference between two samples (color stimuli) defined by $\Delta L^*$, $\Delta a^*$, and $\Delta b^*$. The color difference $\Delta E^*ab$ is defined by the following equation.

$$\Delta E^*ab = [(\Delta L^*)^2 + (\Delta a^*)^2 + (\Delta b^*)^2]^{1/2}$$

The liquid color degradation ($\Delta E^*ab$) during heat sterilization in a packaged tea beverage can be measured using a commercially available spectrophotometric colorimeter.

(Method for suppressing liquid color degradation during heat sterilization of packaged tea beverage)

The method for suppressing liquid color degradation during heat sterilization according to the present invention is not particularly limited as long as the method comprises the step of including lactic acid bacteria in the production of a heat-sterilized packaged tea beverage having a tea polyphenol concentration of 10 to 200 mg/100 mL and a pH of 4.5 to 7.5.

**Examples**

[Test 1] Evaluation of suppression of liquid color degradation by including lactic acid bacteria d - 1

The following test was conducted in order to examine whether liquid color degradation ascribable to the heat sterilization treatment of a tea extract liquid was suppressed by including lactic acid bacteria.

Green tea leaves (Shutoubancha (autumn-winter picking tea)) were placed in hot water of 75°C according to the amount blended described in Table 1, and subjected to solid-liquid separation by extraction for 6 minutes to prepare a green tea extract liquid. To this green tea extract liquid, a dry powder of dead bacteria of a JCM5805 strain, L-ascorbic acid, and baking soda (sodium bicarbonate) were added and mixed according to the amounts blended described in Table 1 to prepare each sample beverage of Test Examples 4 to 6.

In all tests (Tests 1 to 6) of Examples of the present application, the bacterial concentration (hundred-million bacteria/100 mL) of the JCM5805 strain in each beverage was measured using a flow cytometer by appropriately diluting the bacteria with a PBS buffer (manufactured by Takara Bio Inc.), and staining the dilution with thiazole orange using BD Cell Viability Kit with BD Liquid Counting Beads (manufactured by Becton, Dickinson and Company) in accordance with the protocol to prepare a sample. The bacterial concentration (hundred-million bacteria/100 mL) is described as a round number of a 2-digit effective number in tables.

In addition, sample beverages of Test Examples 1 to 3 were also prepared according to the blending described in Table 1.

The sample beverages of Test Examples 2, 3, 5, and 6 were subjected to heat sterilization treatment under the heat treatment conditions described in Table 1.

Black tea leaves were placed in hot water of 85°C according to the amount blended described in Table 1, and subjected to solid-liquid separation by extraction for 6 minutes to prepare a black tea extract liquid. To this black tea extract liquid, a dry powder of dead bacteria of the JCM5805 strain, L-ascorbic acid, and baking soda (sodium bicarbonate) were added and mixed to prepare sample beverages of Test Examples 32 and 33. Sample beverages of Test Examples 30 and 31 were also prepared according to the blending described in Table 1 without adding the dry powder of dead bacteria of the JCM5805 strain. All black tea leaves used in Examples of the present application were produced in Sri Lanka.

The sample beverages of Test Examples 31 and 33 were subjected to heat sterilization treatment under the heat treatment conditions described in Table 1.

The tea polyphenol concentrations of the sample beverages of Test Examples 1 to 6 using green tea leaves and the sample beverages of Test Examples 30 to 33 using black tea leaves were measured by an official method (iron tartrate absorbance method) described in "Explanation of Analytical Manual for Standard Tables of Food Composition in Japan, 5th edition" edited by Japan Food Research Laboratories (Japan Food Research Laboratories ed., Chuohoki Publishing Co., Ltd., July 2001, p. 252). The results are shown in Table 1.

Color difference ΔE*ab after heat sterilization treatment based on the nonheated sample (control) was measured by reflection measurement using a special Petri dish, a white drop lid, and a 20 mm black space ring in a spectrophotometric colorimeter (CM-5 manufactured by Konica Minolta, Inc.). The results are shown in Table 1. In all tests (Tests 1 to 6) of Examples of the present application, the measurement of the color difference ΔE*ab was performed by this method.

[Table 1]

| | Test Example | | | | | | | | | |
|---|---|---|---|---|---|---|---|---|---|---|
| | 1 | 2 | 3 | 30 | 31 | 4 | 5 | 6 | 32 | 33 |
| Green tea leaf (Shutoubancha) (g/100 mL) | 1 | 1 | 1 | - | - | 1 | 1 | 1 | - | - |
| Black tea leaf (g/100 mL) | - | - | - | 1 | 1 | - | - | - | 1 | 1 |
| L-Ascorbic acid (g/100 mL) | 0.04 | 0.04 | 0.04 | 0.04 | 0.04 | 0.04 | 0.04 | 0.04 | 0.04 | 0.04 |
| Baking soda | Added so as to attain pH 6.5 | | | | | | | | | |
| pH | 6.5 | 6.5 | 6.5 | 6.5 | 6.5 | 6.5 | 6.5 | 6.5 | 6.5 | 6.5 |
| Tea polyphenol concentration (mg/100 mL) | 60 | 60 | 60 | 60 | 60 | 60 | 60 | 60 | 60 | 60 |
| JCM5805 strain (hundred-million bacteria/100 mL) | - | - | - | - | - | 900 | 900 | 900 | 900 | 900 |
| Heat treatment conditions | Nonheated | $F_0=4$ | $F_0=20$ | Nonheated | $F_0=20$ | Nonheated | $F_0=4$ | $F_0=20$ | Nonheated | $F_0=20$ |
| Color difference $\Delta E^*ab$ with respect to control | Control | 4.61 | 6.26 | Control | 19.4 | Control | 3.29 | 5.03 | Control | 9.65 |

As is evident from the results of Table 1, when any of green tea leaves and black tea leaves were used, the color difference ΔE*ab (liquid color degradation) with respect to the nonheated sample beverage was more suppressed in the case of including the JCM5805 strain than in the case of not including the JCM5805 strain. Specifically, when green tea leaves were used, the color difference 3.29 of Test Example 5 with respect to the control (Test Example 4) was smaller than the color difference 4.61 of Test Example 2 with respect to the control (Test Example 1). The color difference 5.03 of Test Example 6 with respect to the control (Test Example 4) was smaller than the color difference 6.26 of Test Example 3 with respect to the control (Test Example 1). When black tea leaves were used, the color difference 9.65 of Test Example 33 with respect to the control (Test Example 32) was smaller than the color difference 19.4 of Test Example 31 with respect to the control (Test Example 30).

These results showed that when any of green tea leaves and black tea leaves were used, liquid color degradation ascribable to the heat sterilization treatment of a tea extract liquid was suppressed by including the lactic acid bacteria.

[Test 2] Evaluation of suppression of liquid color degradation by including lactic acid bacteria - 2

The following test was conducted in order to examine how the tea polyphenol concentration of a tea beverage influenced the suppression of liquid color degradation by including lactic acid bacteria.

Green tea leaves (Shutoubancha (autumn-winter picking tea)) were placed in hot water of 75°C according to the amount blended described in Tables 2 to 4, and subjected to solid-liquid separation by extraction for 6 minutes to prepare a green tea extract liquid. To this green tea extract liquid, a dry powder of dead bacteria of a JCM5805 strain, L-ascorbic acid, and baking soda (sodium bicarbonate) were added and mixed according to the amounts blended described in Tables 2 to 4 to prepare each sample beverage of Test Examples 9, 10, 13, 14, 17, and 18.

In addition, sample beverages of Test Examples 7, 8, 11, 12, 15, and 16 containing no dry powder of dead bacteria of the JCM5805 strain were prepared according to the blending described in Tables 2 to 4.

The sample beverages of Test Examples 8, 10, 12, 14, 16, and 18 were subjected to heat sterilization treatment under the heat treatment conditions described in Tables 2 to 4.

The tea polyphenol concentrations of the sample beverages of Test Examples using green tea leaves were measured by the iron tartrate absorbance method mentioned above. The results are shown in Tables 2 to 4.

Color difference ΔE*ab after heat sterilization treatment based on the nonheated sample (control) was measured in a spectrophotometric colorimeter (manufactured by Konica Minolta, Inc.). The results are shown in Tables 2 to 4.

[Table 2]

| | Test Example | | | |
|---|---|---|---|---|
| | 7 | 8 | 9 | 10 |
| Green tea leaf (Shutoubancha) (g/100 mL) | 0.32 | 0.32 | 0.32 | 0.32 |
| L-Ascorbic acid (g/100 mL) | 0.04 | 0.04 | 0.04 | 0.04 |
| Baking soda | Added so as to attain pH 6.5 | | | |
| pH | 6.5 | 6.5 | 6.5 | 6.5 |
| Tea polyphenol concentration (mg/100 mL) | 20 | 20 | 20 | 20 |
| JCM5805 strain (hundred-million bacteria/100 mL) | - | - | 900 | 900 |
| Heat treatment conditions | Nonheated | $F_0$=20 | Nonheated | $F_0$=20 |
| Color difference ΔE*ab with respect to control | Control | 3.34 | Control | 2.86 |

[Table 3]

| | Test Example | | | |
|---|---|---|---|---|
| | 11 | 12 | 13 | 14 |
| Green tea leaf (Shutoubancha) (g/100 mL) | 0.65 | 0.65 | 0.65 | 0.65 |
| L-Ascorbic acid (g/100 mL) | 0.04 | 0.04 | 0.04 | 0.04 |
| Baking soda | Added so as to attain pH 6.5 | | | |
| pH | 6.5 | 6.5 | 6.5 | 6.5 |

(continued)

| | Test Example | | | |
|---|---|---|---|---|
| | 11 | 12 | 13 | 14 |
| Tea polyphenol concentration (mg/100 mL) | 40 | 40 | 40 | 40 |
| JCM5805 strain (hundred-million bacteria/100 mL) | - | - | 900 | 900 |
| Heat treatment conditions | Nonheated | $F_0=20$ | Nonheated | $F_0=20$ |
| Color difference ΔE*ab with respect to control | Control | 5.18 | Control | 4.77 |

[Table 4]

| | Test Example | | | |
|---|---|---|---|---|
| | 15 | 16 | 17 | 18 |
| Green tea leaf (Shutoubancha) (g/100 mL) | 2.4 | 2.4 | 2.4 | 2.4 |
| L-Ascorbic acid (g/100 mL) | 0.04 | 0.04 | 0.04 | 0.04 |
| Baking soda | Added so as to attain pH 6.5 | | | |
| pH | 6.5 | 6.5 | 6.5 | 6.5 |
| Tea polyphenol concentration (mg/100 mL) | 150 | 150 | 150 | 150 |
| JCM5805 strain (hundred-million bacteria/100 mL) | - | - | 900 | 900 |
| Heat treatment conditions | Nonheated | $F_0=20$ | Nonheated | $F_0=20$ |
| Color difference ΔE*ab with respect to control | Control | 10.09 | Control | 7.35 |

As is evident from the results of Tables 2 to 4, at all the tea polyphenol concentrations, the color difference ΔE*ab (liquid color degradation) with respect to the nonheated sample beverage was more suppressed in the case of including the JCM5805 strain than in the case of not including the JCM5805 strain.

[Test 3] Evaluation of suppression of liquid color degradation by including lactic acid bacteria - 3

The following test was conducted in order to examine how the amount of lactic acid bacteria blended influenced the suppression of liquid color degradation by including the lactic acid bacteria.
Green tea leaves (Shutoubancha (autumn-winter picking tea)) were placed in hot water of 75°C according to the amount blended described in Table 5, and subjected to solid-liquid separation by extraction for 6 minutes to prepare a green tea extract liquid. To this green tea extract liquid, a dry powder of dead bacteria of a JCM5805 strain, L-ascorbic acid, and baking soda (sodium bicarbonate) were added and mixed according to the amounts blended described in Table 5 to prepare each sample beverage of Test Examples 19 to 23 and 34.
The sample beverages of Test Examples 19 to 23 and 34 were subjected to heat sterilization treatment under the heat treatment conditions described in Table 5.
The tea polyphenol concentrations of the sample beverages of Test Examples using green tea leaves were measured by the iron tartrate absorbance method mentioned above. The results are shown in Table 5.
Color difference ΔE*ab after heat sterilization treatment based on the nonheated sample (control) was measured in a spectrophotometric colorimeter (manufactured by Konica Minolta, Inc.). The results are shown in Table 5.

[Table 5]

| | Test Example | | | | | |
|---|---|---|---|---|---|---|
| | 34 | 19 | 20 | 21 | 22 | 23 |
| Green tea leaf (Shutoubancha) (g/100 mL) | 1 | 1 | 1 | 1 | 1 | 1 |
| L-Ascorbic acid (g/100 mL) | 0.04 | 0.04 | 0.04 | 0.04 | 0.04 | 0.04 |
| Baking soda | Added so as to attain pH 6.5 | | | | | |

(continued)

| | Test Example | | | | | |
|---|---|---|---|---|---|---|
| | 34 | 19 | 20 | 21 | 22 | 23 |
| pH | 6.5 | 6.5 | 6.5 | 6.5 | 6.5 | 6.5 |
| Tea polyphenol concentration (mg/100 mL) | 60 | 60 | 60 | 60 | 60 | 60 |
| JCM5805 strain (hundred-million bacteria/100 mL) | 100 | 150 | 300 | 900 | 1500 | 4500 |
| Heat treatment conditions | $F_0$=20 | $F_0$=20 | $F_0$=20 | $F_0$=20 | $F_0$=20 | $F_0$=20 |
| Color difference ΔE*ab with respect to control | 6.10 | 5.83 | 5.12 | 5.03 | 5.07 | 3.22 |

In Table 5, all the numeric values of the color difference ΔE*ab in Test Examples 19 to 23 and 34 with respect to the nonheated sample were smaller than color difference ΔE*ab 6.26 of the nonheated sample (Test Example 3 of Table 1) in the case of not including the JCM5805 strain, showing that liquid color degradation was suppressed in all of Test Examples 19 to 23 and 34. As is evident from the results of Table 5, it was shown that the color difference ΔE*ab (liquid color degradation) with respect to the nonheated sample beverage was more largely suppressed with increase in the amount of the JCM5805 strain contained.

[Test 4] Evaluation of suppression of liquid color degradation by including lactic acid bacteria - 4

The following test was conducted in order to examine whether liquid color degradation during heat sterilization could be suppressed by including lactic acid bacteria in the case of using green tea leaves of different type from the green tea leaves (Shutoubancha (autumn-winter picking tea)) used in Tests 1 to 3.
Green tea leaves (Nibancha (second picking tea)) were placed in hot water of 75°C according to the amount blended described in Table 6, and subjected to solid-liquid separation by extraction for 6 minutes to prepare a green tea extract liquid. To this green tea extract liquid, a dry powder of dead bacteria of a JCM5805 strain, L-ascorbic acid, and baking soda (sodium bicarbonate) were added and mixed according to the amounts blended described in Table 6 to prepare a sample beverage of Test Example 25.
In addition, a sample beverage of Test Example 24 containing no dry powder of dead bacteria of the JCM5805 strain was prepared according to the blending described in Table 6.
The sample beverages of Test Examples 24 and 25 were subjected to heat sterilization treatment under the heat treatment conditions described in Table 6.
The tea polyphenol concentrations of the sample beverages of Test Examples using green tea leaves were measured by the iron tartrate absorbance method mentioned above. The results are shown in Table 6.
Color difference ΔE*ab after heat sterilization treatment based on the nonheated sample (control) was measured in a spectrophotometric colorimeter (manufactured by Konica Minolta, Inc.). The results are shown in Table 6.

[Table 6]

| | Test Example | |
|---|---|---|
| | 24 | 25 |
| Green tea leaf (Nibancha) (g/100 mL) | 0.8 | 0.8 |
| L-Ascorbic acid (g/100 mL) | 0.04 | 0.04 |
| Baking soda | Added so as to attain pH 6.5 | |
| pH | 6.5 | 6.5 |
| Tea polyphenol concentration (mg/100 mL) | 60 | 60 |
| JCM5805 strain (hundred-million bacteria/100 mL) | - | 900 |
| Heat treatment conditions | $F_0$=20 | $F_0$=20 |
| Color difference ΔE*ab with respect to control | 14.79 | 8.40 |

As is evident from the results of Table 6, when Nibancha (second picking tea) was used as tea leaves, the color difference ΔE*ab (liquid color degradation) with respect to the nonheated sample beverage was also more suppressed in the case of including the JCM5805 strain than in the case of not including the JCM5805 strain.

[Test 5] Evaluation of suppression of liquid color degradation by including lactic acid bacteria - 5

In Tests 1 to 4, a batch-type retort sterilization apparatus was used. The following test was conducted in order to examine whether liquid color degradation during heat sterilization could also be suppressed by including lactic acid bacteria in the case of using a continuous UHT sterilization apparatus, and whether liquid color degradation during heat sterilization could also be suppressed by including lactic acid bacteria in the case of using green tea leaves and dust tea in combination, for example.

Green tea leaves (Shutoubancha (autumn-winter picking tea); the same product as the Shutoubancha used in Tests 1 to 3) were placed in hot water of 75°C according to the amount blended described in Table 7, and subjected to solid-liquid separation by extraction for 6 minutes to prepare a green tea extract liquid. To this green tea extract liquid, a dry powder of dead bacteria of a JCM5805 strain, L-ascorbic acid, and baking soda (sodium bicarbonate) were added and mixed according to the amounts blended described in Table 7 to prepare a sample beverage of Test Example 27.

A sample beverage of Test Example 26 containing no dry powder of dead bacteria of the JCM5805 strain was prepared according to the blending described in Table 7.

Green tea leaves (Shutoubancha (autumn-winter picking tea) B; a different product from the Shutoubancha used in Tests 1 to 3) were placed in hot water of 75°C according to the amount blended described in Table 7, and subjected to solid-liquid separation by extraction for 6 minutes to prepare a green tea extract liquid. To this green tea extract liquid, dust tea, a dry powder of dead bacteria of a JCM5805 strain, L-ascorbic acid, and baking soda (sodium bicarbonate) were added and mixed according to the amounts blended described in Table 7 to prepare a sample beverage of Test Example 29.

A sample beverage of Test Example 28 containing no dry powder of dead bacteria of the JCM5805 strain was prepared according to the blending described in Table 7.

The sample beverages of Test Examples 26 to 29 were subjected to heat sterilization treatment in a UHT sterilization apparatus under the heat treatment conditions described in Table 7.

The tea polyphenol concentration of the sample beverage of each Test Example using green tea leaves was measured by the iron tartrate absorbance method mentioned above. The results are shown in Table 7.

Color difference ΔE*ab after heat sterilization treatment based on the nonheated sample (control) was measured in a spectrophotometric colorimeter (manufactured by Konica Minolta, Inc.). The results are shown in Table 7.

[Table 7]

| | Test Example | | | |
|---|---|---|---|---|
| | 26 | 27 | 28 | 29 |
| Green tea leaf (Shutoubancha) (g/100 mL) | 1 | 1 | - | - |
| Green tea leaf (Shutoubancha B) (g/100 mL) | - | - | 0.8 | 08 |
| Dust tea (g/100 mL) | - | - | 0.025 | 0.025 |
| L-Ascorbic acid (g/100 mL) | 0.04 | 0.04 | 0.04 | 0.04 |
| Baking soda | Added so as to attain pH 6.5 | | Added so as to attain pH 7 | |
| pH | 6.5 | 6.5 | 7 | 7 |
| Tea polyphenol concentration (mg/100 mL) | 60 | 60 | 50 | 50 |
| JCM5805 strain (hundred-million bacteria/100 mL) | - | 900 | - | 300 |
| Heat treatment conditions | $F_0=20$ | $F_0=20$ | $F_0=20$ | $F_0=20$ |
| Color difference ΔE*ab with respect to control | 3.58 | 2.06 | 3.52 | 1.94 |

As is evident from the results of Table 7, when the continuous UHT sterilization apparatus was used, when green tea leaves and dust tea were used in combination, or when pH was adjusted to 7, for example, the color difference ΔE*ab (liquid color degradation) with respect to the nonheated sample beverage was also more suppressed in the case of including the JCM5805 strain than in the case of not including the JCM5805 strain.

[Test 6] Evaluation of suppression of liquid color degradation by including lactic acid bacteria - 6

The following test was conducted in order to examine how the amount of lactic acid bacteria blended influenced the suppression of liquid color degradation by including the lactic acid bacteria in the case of a black tea beverage.

Black tea leaves were placed in hot water of 85°C according to the amount blended described in Table 8, and subjected to solid-liquid separation by extraction for 6 minutes to prepare a black tea extract liquid. To this black tea extract liquid, a dry powder of dead bacteria of a JCM5805 strain, L-ascorbic acid, and baking soda (sodium bicarbonate) were added and mixed according to the amounts blended described in Table 8 to prepare each sample beverage of Test Examples 35 to 37.

The sample beverages of Test Examples 35 to 37 were subjected to heat sterilization treatment under the heat treatment conditions described in Table 8.

The tea polyphenol concentrations of the sample beverages of Test Examples 35 to 37 using black tea leaves were measured by the iron tartrate absorbance method mentioned above. The results are shown in Table 8.

Color difference $\Delta E^*ab$ after heat sterilization treatment based on the nonheated sample (control) was measured in a spectrophotometric colorimeter (manufactured by Konica Minolta, Inc.). The results are shown in Table 8.

[Table 8]

|  | Test Example | | |
| --- | --- | --- | --- |
|  | 35 | 36 | 37 |
| Black tea leaf (g/100 mL) | 1 | 1 | 1 |
| L-Ascorbic acid (g/100 mL) | 0.04 | 0.04 | 0.04 |
| Baking soda | Added so as to attain pH 6.5 | | |
| pH | 6.5 | 6.5 | 6.5 |
| Tea polyphenol concentration (mg/100 mL) | 60 | 60 | 60 |
| JCM5805 strain (hundred-million bacteria/100 mL) | 100 | 300 | 600 |
| Heat treatment conditions | $F_0=20$ | $F_0=20$ | $F_0=20$ |
| Color difference $\Delta E^*ab$ with respect to control | 16.1 | 16.1 | 11.9 |

In Table 8, all the numeric values of the color difference $\Delta E^*ab$ in Test Examples 35 to 37 with respect to the nonheated sample were smaller than color difference $\Delta E^*ab$ 19.4 of the nonheated sample (Test Example 31 of Table 1) in the case of not including the JCM5805 strain, showing that liquid color degradation was suppressed in all of Test Examples 35 to 37. As is evident from the results of Table 8, it was shown that in the black tea beverage as well, the color difference $\Delta E^*ab$ (liquid color degradation) with respect to the nonheated sample beverage was more largely suppressed with increase in the amount of the JCM5805 strain contained.

**Claims**

1. A heat-sterilized packaged tea beverage having a tea polyphenol concentration of 10 to 200 mg/100 mL and a pH of 4.5 to 7.5, wherein the packaged tea beverage comprises lactic acid bacteria.

2. The packaged tea beverage according to claim 1, wherein a content of the lactic acid bacteria is 10 billion or more bacteria/100 mL.

3. The packaged tea beverage according to claim 1 or 2, wherein the lactic acid bacteria are bacteria of the genus *Lactococcus*.

4. The packaged tea beverage according to any one of claims 1 to 3, wherein the lactic acid bacteria are *Lactococcus lactis* subsp. *lactis* JCM5805.

5. The packaged tea beverage according to any one of claims 1 to 4, wherein the packaged tea beverage is a packaged green tea beverage or a packaged black tea beverage.

6. A method for producing a packaged tea beverage, comprising a step of including lactic acid bacteria in the production of a heat-sterilized packaged tea beverage having a tea polyphenol concentration of 10 to 200 mg/100 mL and a pH of 4.5 to 7.5.

7. The method for producing a packaged tea beverage according to claim 6, wherein the step of including lactic acid bacteria is carried out at any stage before heat sterilization in the production of the packaged tea beverage.

8. A method for suppressing liquid color degradation during heat sterilization of a packaged tea beverage, comprising a step of including lactic acid bacteria, in the production of a heat-sterilized packaged tea beverage having a tea polyphenol concentration of 10 to 200 mg/100 mL and a pH of 4.5 to 7.5.

9. The method for suppressing liquid color degradation during heat sterilization of a packaged tea beverage according to claim 8, wherein the step of including lactic acid bacteria is carried out at any stage before heat sterilization, in the production of the packaged tea beverage.

[Fig. 1]

| INTERNATIONAL SEARCH REPORT | International application No. |
|---|---|
| | **PCT/JP2022/011200** |

**A.    CLASSIFICATION OF SUBJECT MATTER**

*A23F 3/16*(2006.01)i; *C12N 1/20*(2006.01)i
FI:    A23F3/16; C12N1/20 A

According to International Patent Classification (IPC) or to both national classification and IPC

**B.    FIELDS SEARCHED**

Minimum documentation searched (classification system followed by classification symbols)

A23F3/16; C12N1/20

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); FSTA (STN)

**C.    DOCUMENTS CONSIDERED TO BE RELEVANT**

| Category* | Citation of document, with indication, where appropriate, of the relevant passages | Relevant to claim No. |
|---|---|---|
| X | JP 2013-220055 A (KIRIN BEVERAGE CORP.) 28 October 2013 (2013-10-28) | 1, 5-7 |
| | claims 1, 5, paragraph [0008], tables 1-2, 5, test examples 4-5 | |
| A | claims 1, 5, paragraph [0008], tables 1-2, 5, test examples 4-5 | 2-4, 8-9 |
| X | WO 2006/028164 A1 (KIRIN BEER KK) 16 March 2006 (2006-03-16) | 1-3, 5-7 |
| | claims 13-17, examples 1-3, table 1, paragraphs [0001], [0027] | |
| A | claims 13-17, examples 1-3, table 1, paragraphs [0001], [0027] | 4, 8-9 |
| X | JP 2019-103408 A (ASAHI SOFT DRINKS CO., LTD.) 27 June 2019 (2019-06-27) | 1-3, 5-7 |
| | paragraphs [0001], [0026], [0062], [0073], experimental example 3, table 5 | |
| Y | paragraphs [0001], [0026], [0062], [0073], experimental example 3, table 5 | 4-5, 8-9 |
| A | JP 2017-195782 A (KIRIN CO., LTD.) 02 November 2017 (2017-11-02) | 1-3, 6-7 |
| | paragraph [0049] | |
| Y | paragraph [0049] | 4-5, 8-9 |

☑ Further documents are listed in the continuation of Box C.          ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **30 March 2022** | **12 April 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT

| International application No. |
| --- |
| **PCT/JP2022/011200** |

**C.    DOCUMENTS CONSIDERED TO BE RELEVANT**

| Category* | Citation of document, with indication, where appropriate, of the relevant passages | Relevant to claim No. |
| --- | --- | --- |
| A | JP 2007-254313 A (ASAHI BREWERIES, LTD.) 04 October 2007 (2007-10-04) paragraph [0041] | 1-3, 6-7 |
| Y | paragraph [0041] | 4-5, 8-9 |
| Y | 藤原大介, 城内健太, 杉村哲, ウイルス感染防御を統括するプラズマサイトイド樹状細胞を活性化する乳酸菌の開発, 化学と生物, 2015, vol. 53, no. 9, pages 626-632, non-official translation (FUJIWARA, Daisuke, JOUNAI, Kenta, SUGIMURA, Tetsu. Development of lactic acid bacteria that activate plasmacytoid dendritic cells that control virus infection defense. Kagaku to seibutsu.) particularly, "In conclusion" | 4-5 |
| A | particularly, "In conclusion" | 1-3, 6-9 |
| Y | JP 2006-217914 A (SK FOODS KK) 24 August 2006 (2006-08-24) paragraph [0001], examples, application example C3 | 8-9 |
| A | paragraph [0001], examples, application example C3 | 1-7 |

Form PCT/ISA/210 (second sheet) (January 2015)

**INTERNATIONAL SEARCH REPORT**
**Information on patent family members**

International application No.

**PCT/JP2022/011200**

| Patent document cited in search report | | | Publication date (day/month/year) | Patent family member(s) | Publication date (day/month/year) |
|---|---|---|---|---|---|
| JP | 2013-220055 | A | 28 October 2013 | (Family: none) | |
| WO | 2006/028164 | A1 | 16 March 2006 | US 2008/0096266 A1 claims 13-17, examples 1-3, table 1, paragraphs [0001], [0032] | |
| JP | 2019-103408 | A | 27 June 2019 | (Family: none) | |
| JP | 2017-195782 | A | 02 November 2017 | (Family: none) | |
| JP | 2007-254313 | A | 04 October 2007 | (Family: none) | |
| JP | 2006-217914 | A | 24 August 2006 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 1571801 A
- JP 2001112414 A
- JP 2007060972 A
- JP 2008005811 A
- JP 2019122316 A

**Non-patent literature cited in the description**

- Explanation of Analytical Manual for Standard Tables of Food Composition in Japan. Chuohoki Publishing Co., Ltd, July 2001, 252